(19) **Europäisches Patentamt / European Patent Office / Office européen des brevets**

(11) **EP 3 050 560 B1**

(12) # EUROPEAN PATENT SPECIFICATION

(45) Date of publication and mention
of the grant of the patent:
**08.01.2025 Bulletin 2025/02**

(21) Application number: **14848881.0**

(22) Date of filing: **24.09.2014**

(51) International Patent Classification (IPC):
**A61K 9/20** *(2006.01)*    **A61K 31/166** *(2006.01)*
**A61K 31/167** *(2006.01)*   **A61K 31/192** *(2006.01)*
**A61K 31/198** *(2006.01)*   **A61K 31/375** *(2006.01)*
**A61K 31/4164** *(2006.01)*  **A61K 31/426** *(2006.01)*
**A61K 47/38** *(2006.01)*    **A61P 1/04** *(2006.01)*
**A61P 3/02** *(2006.01)*     **A61P 29/00** *(2006.01)*
**A61P 31/10** *(2006.01)*

(52) Cooperative Patent Classification (CPC):
**A61K 31/167; A61K 9/0056; A61K 31/198;
A61K 31/375; A61K 31/4174; A61K 31/426;
A61P 1/04; A61P 3/02; A61P 29/00; A61P 31/10**

(86) International application number:
**PCT/JP2014/075228**

(87) International publication number:
**WO 2015/046219 (02.04.2015 Gazette 2015/13)**

(54) **INTRAORALLY DISINTEGRABLE TABLET COMPRISING DISINTEGRABLE GRANULAR COMPOSITION**

INTRAORAL ZERFALLENDE TABLETTE MIT EINER ZERFALLENDEN GRANULATZUSAMMENSETZUNG

COMPRIMÉ POUVANT SE DÉSINTÉGRER PAR VOIE INTRA-ORALE COMPRENANT UNE COMPOSITION GRANULAIRE POUVANT SE DÉSINTÉGRER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2013 JP 2013202247**

(43) Date of publication of application:
**03.08.2016 Bulletin 2016/31**

(73) Proprietor: **Daicel Corporation
Osaka-shi, Osaka 530-0011 (JP)**

(72) Inventors:
• **HIRAMURA, Takahiro**
  **Tokyo 108-8230 (JP)**
• **IKURA, Kiyoshi**
  **Himeji-shi**
  **Hyogo 671-1281 (JP)**
• **ITAYA, Sae**
  **Himeji-shi**
  **Hyogo 671-1281 (JP)**

• **OKABAYASHI, Tomohito**
  **Himeji-shi**
  **Hyogo 671-1281 (JP)**
• **SAKAGUCHI, Anan**
  **Himeji-shi**
  **Hyogo 671-1281 (JP)**
• **MORITA, Tetsuro**
  **Itami-shi**
  **Hyogo 664-0898 (JP)**
• **IKEDA, Kimiko**
  **Itami-shi**
  **Hyogo 664-0898 (JP)**

(74) Representative: **J A Kemp LLP
80 Turnmill Street
London EC1M 5QU (GB)**

(56) References cited:
**EP-A1- 1 923 074      EP-A1- 2 465 539
EP-A1- 2 832 350      EP-A1- 2 898 899
EP-B1- 2 465 539      EP-B1- 2 832 350
EP-B1- 2 898 899      WO-A1-2010/061846**

EP 3 050 560 B1

WO-A1-2013/146917    JP-A- 2007 056 011
JP-A- 2012 031 138    JP-A- 2013 147 470

## Description

Technical Field

The present disclosure relates to an orally disintegrating tablet comprising a disintegrative particulate composition, particularly that comprising an acid-type carboxymethylcellulose and obtainable by the production of a multi-stage granulation process.

Background Art

In the past, orally disintegrating tablets have been developed as highly convenient forms which can safely be taken by patients who have difficulty in swallowing drugs, elderly people, children, etc., and which can easily be taken without water. It is important that the orally disintegrating tablets have sufficient breaking strength (tablet hardness) such that any cracks, powdering, etc. are not caused in the tablets during production or transportation of the tablets or during breaking their seals in the same manner as general tablets, and also, it is important that the orally disintegrating tablets have excellent disintegrability (disintegration time) such that the tablets immediately disintegrate in the oral cavity.

An excellent moldability will be required in the production of a tablet. The moldability means the relation between a tablet compression force and the tablet hardness obtained thereby. A process that needs a high tablet compression force could cause problems such as limitation in the performance of a tablet-compressing machine, reduction of productivity and reduction in the properties of coating particles comprised in the tablet. It will be therefore important for the particle or particulate composition constituting the tablet to have such an excellent moldability that a higher tablet hardness can be obtained with the same tablet compression force, or that the same tablet hardness can be obtained by a lower tablet compression force.

The tablet hardness and disintegrability are mutually opposing properties. In general, when a molding pressure is increased to increase the hardness, the disintegration time will tend to be prolonged, and, when the molding pressure is reduced to shorten the disintegration time, the hardness will tend to be smaller. Therefore, various technologies have been developed in order to cope with both the two properties or to achieve an optimal balance between the two properties.

Furthermore, the components of particles, granulation methods, etc. have been studied in order to impart superior moldability to the particles or particulate compositions constituting tablets.

An acid-type carboxymethylcellulose, which is a cellulose derivative called "carmellose", will swell when mixed with water and become a suspension with little viscosity. It is comprised in the orally disintegrating tablet as a component such as a substrate, binder, excipient or disintegrator.

Crystalline cellulose is a water-insoluble substance in white powder that is obtained by a partial depoymerization and purification of $\alpha$-cellulose derived from fibrous plant. As this substance is tasteless and chemically stable, it is used as a pharmaceutical additive, especially as an excipient, binder or disintegrator in the formulation of a tablet. It is also used in cosmetics, dairy products and the like as an emulsion-stabilizing agent, etc.

For example, Patent Literature (PTL) 1 discloses a disintegrative particulate composition that is produced by homogeneously dispersing mannitol, xylitol, inorganic excipient, disintegrator and carmellose in the presence of water, followed by drying. The composition is characterized in that xylitol is solid-dispersed in mannitol particles to form composite particles, and that inorganic excipient, disintegrator and carmellose are dispersed in the composite particles. Said disintegrative particulate composition is produced by spray-granulation of the dispersion wherein the above components are dispersed in aqueous solvent or by spraying to carriers made of mannitol and the like.

PTL 2 discloses an orally disintegrating tablet comprising carboxymethylcellulose in an amount of 10% (w/w) or more based on the total amount including medicinal ingredients. It is produced by mixing each component, followed by formulation with a tableting machine.

PTL 3 discloses a method for the production of an orally disintegrating tablet comprising loratadine as a medicinal ingredient. The method include two granulation steps, wherein loratadine is granulated with at least one of additives such as a binder, excipient, disintegrator and the like in a first granulation step, and the resulting granules obtained in the first granulation step is further granulated with at least one of the same additives as in the first step. Carmellose is listed as an example of the disintegrator.

Furthermore, PTL 4 discloses a method for the production of an orally disintegrating tablet. The method comprises a step of spraying an aqueous suspension of a water-soluble and hydrophilic disintegrator onto a mixture of a medicinal ingredient and an excipient to give a granulate (A) comprising the medicinal ingredient, and a step of spraying an aqueous suspension of the same water-soluble and hydrophilic disintegrator as above onto the excipient to give a granulate (B) without the medicinal ingredient, and a step of compression molding of the granulates (A) and (B).

Related Arts

Patent Literatures

PTL 1: International Publication Pamphlet WO2011/019045
PTL 2: JP-A-2008-285434
PTL 3: JP-A-2012-31138
PTL 4: Specification of Japanese Patent No. 4551627

The following documents are also mentioned:

EP 2,832,350 A1 which is concerned with a method for producing a disintegrating particulate composition, the disintegrating particulate composition and orally
disintegrating tablets comprising the composition;
EP 2,898,899 A1 which is concerned with a disintegrating particulate composition and orally disintegrating tablets containing the composition;
EP 2,465,539 A1 which is concerned with a disintegrating tablets comprising the composition;
EP 2,898,899 A1 which is concerned with a disintegrating particulate composition and orally disintegrating tablets containing the composition;
EP 2,465,539 A1 which is concerned with a disintegrating particle composition and orally rapidly disintegrating tablet; and
EP 1,923,074 A1 which is concerned with an orally disintegratable tablet.

Summary of Invention

Problems to be solved by the Invention

It has been desired that a content of a medicinal disintegrating particle composition and orally rapidly disintegrating tablet; and
EP 1,923,074 A1 which is concerned with an orally disintegratable tablet.

Summary of Invention

Problems to be solved by the Invention

It has been desired that a content of a medicinal ingredient is increased in an orally disintegrating tablet. However, if said content is increased, it will cause problems such as that it will be difficult to obtain a desired tablet hardness, and that a high tablet compression force necessary to increase the tablet hardness will lengthen the disintegration time.
Accordingly, an object of the present invention is to solve such technical problems as mentioned above, and to provide an orally disintegrating tablet having both an excellent tablet hardness and disintegrability even when the content of a medicinal ingredient is high.

Means to Solve the Problem

The present inventors have earnestly studied and found that an orally disintegrating tablet with a high content of a medicinal ingredient and having both an excellent tablet hardness and disintegrability can be produced by using an medicinal ingredient having distribution coefficient (logP) in a range of from -6.0 to 10.0 with a disintegrative particulate composition produced by granulation consisting of at least two stages and comprising an acid-type carboxymethylcellulose as a first disintegrator.
Thus, the present invention relates to an orally disintegrating tablet comprising a medicinal ingredient having distribution coefficient (logP) in a range of from - 5.0 to 6.0 and a disintegrative particulate composition,

wherein the content of the medicinal ingredient is 40% by weight or more;
wherein the disintegrative particulate composition includes four components consisting of a first disintegrator component of an acid-type carboxymethylcellulose, a second disintegrator component other than the acid-type carboxymethylcellulose, an excipient of sugars or sugar alcohols, and crystalline cellulose;

4

wherein the disintegrative particulate composition is produced by a two-stage granulation comprising a first wet granulation step using any two or three of the four components, and a second wet granulation step using at least granules obtained in the first wet granulation step and the remaining one or two of the four components not used in the first wet granulation step; and

wherein the distribution coefficient is calculated according to the formulation (1) when the medicinal ingredient does not have a structure of a zwitterion, and to formulation (2) when the medicinal ingredient has a structure of a zwitterion:

(1) CAChe using the following equation:

$$LogP = \Sigma niai$$

wherein "ni" means the number of each group of atoms, and "ai" means a contribution ratio of the group of atoms;
(2) KOWWIN using the following equation:

$$LogP = \Sigma fini + \Sigma cjnj + 0.229$$

wherein "fi" means the coefficient of each group of atoms, and "ni" means the number of the group of atoms, and "cj" means the coefficient of each correction factor, and "nj" means the number of the correction factor.

Advantageous Effects of Invention

According to the present invention, it has become possible to provide an orally disintegrating tablet with a high content of a medicinal ingredient and having both an excellent tablet hardness and disintegrability.

Concise Explanation of Drawings

Fig. 1 shows correlation between a value of the distribution coefficient (logP) calculated by CAChe and an experimental value.
Fig. 2 shows correlation between a value of the distribution coefficient (logP) calculated by KOWWIN and an experimental value.

Description of Embodiments

The present disclosure relates to the orally disintegrating tablet comprising a medicinal ingredient having distribution coefficient (logP) in a range of from -6.0 to 10.0, preferably of from -5.0 to 6.0, and a disintegrative particulate composition produced by granulation consisting of at least two stages.

The distribution coefficient is a numerical value showing hydrophobicity of a compound as a whole. The distribution coefficient of each medicinal ingredient in the present invention is calculated as follows.

Thus, values obtained according to the formulation (1) were principally adopted as a calculated one, and values obtained according to the formulation (2) were adopted as a calculated one when the medicinal ingredient has a structure of a zwitterion such as an amino acid.

(1) The value of logP calculated by CAChe using the following equation:

$$LogP = \Sigma n_i a_i$$

Wherein $n_i$ means the number of each group of atoms, and $a_i$ means a contribution ratio of the group of atoms. A numerical value of the contribution ratio may be referred to the following article.
(Reference Article: Journal of Computational Chemistry, Vol.9, No. 1, 80-90 (1988))
(2) The value of logP calculated by KOWWIN using the following equation:

$$LogP = \Sigma f_i n_i + \Sigma c_j n_j + 0.229$$

Wherein $f_i$ means the coefficient of each group of atoms, and $n_i$ means the number of the group of atoms, and $c_j$ means the coefficient of each correction factor, and $n_j$ means the number of the correction factor. A numerical value of the coefficient of each group of atoms and the coefficient of each correction factor may be referred to the following article.

(Reference Article: Journal of Pharmacological Science, 84, 83-92 (1995))
Thus, it is estimated that water is hardly conducted deeply into the inside of an orally disintegrating tablet comprising a medicinal ingredient with a high distribution coefficient (high hydrophobicity) so that disintegrability of the tablet can be damaged. On the other hand, it is considered that a medicinal ingredient with a low distribution coefficient (high hydrophilicity), which is easily dissolved in water, will increase the binding between the medicinal ingredients comprised in a tablet when the tablet is compressed. As a result, the moldability and disintegrability of the tablet can be reduced.

On the other hand, the measured value of the distribution coefficient may be calculated by the following equation using a concentration of a medicinal ingredient dissolved in each of two phases of water and n-octanol, which is obtained by a conventional quantitative method such as absorption spectrophotometry, gas chromatography or liquid chromatography:

LogP = Log[(concentration of the medicinal ingredient in n-octanol phase)/( concentration of the medicinal ingredient in water phase)

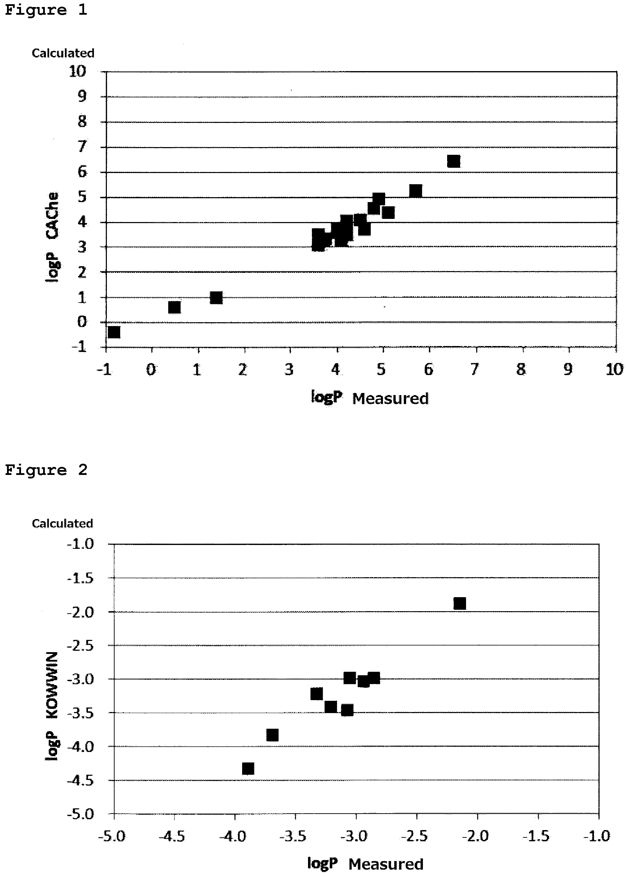

The relation between the calculated values and the measured (experimental) values are shown in Figs 1 and 2.

The medicinal ingredient comprised in the orally disintegrating tablet according to the present invention includes a pharmaceutical ingredient, and nutrient ingredient comprised in foods and health food products. The medicinal ingredient may be added as such or in a coated or granulated form for the purpose of sustained release or masking of bitterness of the medicinal ingredient.

There is no limitation on an application or kind of the medicinal ingredients comprised in the orally disintegrating tablet according to the present invention, which may include, for example, agents affecting each organ such as the central nervous system, peripheral nervous system, a sensory organ, a circulatory organ, a respiratory organ and a digestive organ and an urogenital organ; hormone drug; agents affecting metabolism such as a vitamin drug, an analeptic, an agent affecting blood and body fluid; agents affecting the function of tissue and cell such as an agent activating cellular function, an agent affecting tumors, an radioactive medicine, an anti-allergic agent; medicines based on a medical prescription relating to herbal medicines and Chinese medicines; antibiotics; agents for chemotherapy, biological drug; agents for pathogenic organisms such as parasites; agents for formulation use, diagnosis, public health and in-vitro diagnosis.

The medicinal ingredient that satisfies the above particular range of the distribution coefficient is selected from the group consisting of clotrimazole, ibuprofen, ethenzamide, acetaminophen, famotidine, ascorbic acid, glycine, loxoprofen, caffeine, loratadine, aldioxa, pipemidic acid, afloqualone, clofibrate, and aspartic acid. Table 1 below shows the caluculated and experimental values of the distribution coefficients of the representative examples of the medicinal ingredients used in the present invention

[TABLE 1]

| Medicinal Ingredient | logP Observed | CAChe Calculated | KOWWIN Calculated |
|---|---|---|---|
| clotrimazole | | 5.19 | |
| ibuprofen | 3.5 | 3.83 | |
| ethenzamide | 1.39 | 0.97 | |
| acetaminophen | 0.49 | 0.61 | |
| famotidine | -0.82 | -0.42 | |
| ascorbic acid | -2.15 | | -1.88 |
| glycine | -3.21 | | -3.41 |
| aspartic acid | -3.89 | | -4.32 |

Although there is no limitation in the content of the medicinal ingredient comprised in the orally disintegrating tablet according to the present invention, it may comprise a content of the medicinal ingredient preferably of 40% or more, or more preferably of 50% or more.

Thus, the orally disintegrating tablet according to the present invention may comprise the content of the medicinal ingredient of 60% or more, which has the distribution coefficient (logP) in a range of from 0.8 to 4.0 and.

The orally disintegrating tablet according to the present invention may optionally include other pharmaceutically-acceptable components as additives such as excipients, surfactants, lubricants, acidulants, sweeteners, corrigents, flavoring agents, colorants, and stabilizing agents, when needed. As these optional components, for example, appropriate ingredients described in "Japanese Pharmaceutical Excipients Directory" (YAKUJI NIPPO LIMITED) or the Japanese Pharmacopoeia can be used. Also, the types and blending ratios of each optional ingredient (component) are not particularly limited as long as the expected effects of the present invention are brought about, and the blending ratios can properly be determined by those skilled in the art. The orally disintegrating tablet can be formulated by any methods known to those skilled in the art, for example, by tableting.

Four mechanisms of "wicking", "swelling", "deformation" and "repulsion" have been proposed as mechanisms of disintegration of tablets or the like. Among them, "wicking" is a disintegration mechanism which proceeds upon weakened binding force between particles included in the tablet as a result of water permeation through components such as disintegrators included in the tablet. As a typical example of a disintegrator having a higher effect to promote such "wicking", an acid-type carboxymethylcellulose has been known. Also, "swelling" is a disintegration mechanism which proceeds upon swelling of disintegrators themselves as a result of water permeation through the disintegrators.

The acid-type carboxymethylcellulose, which is the first disintegrator component included in the disintegrative particulate composition produced by granulation consisting of at least two stages according to the present invention, is a substance called carmellose, and has been used as a pharmaceutical additive. In the same manner as the acid-type carboxymethylcellulose, for example, both calcium salt of carboxymethylcellulose and a cross-linked product of carboxymethylcellulose sodium are water-insoluble, and have been used as disintegrator for tablets, etc. On the other hand, sodium salt of carboxymethylcellulose is water-soluble, and has been used for purposes of a binder, etc. In addition, in some cases, a salt of carboxymethylcellulose may be referred to as carmellose.

For the second disintegrator component of the disintegrative particulate composition of the present invention, any disintegrators other than the acid-type carboxymethylcellulose, which have been known to a person skilled in the art, can be used. However, in order to obtain combined effects of the different disintegration mechanisms as shown above, it is preferable that a disintegrator having a superior effect to promote a mechanism other than "wicking" (e.g. "swelling") be used as the second disintegrator component. Suitable examples of such a disintegrator include crospovidone, croscarmellose sodium, carboxymethyl starch sodium, low substituted hydroxypropylcellulose, carboxymethylcellulose calcium, hydroxypropyl starch, and starch. Additionally, crospovidone is a common name for a cross-linked polymer of 1-vinyl-2-pyrrolidone, and croscarmellose sodium is a common name for a cross-linked product of carboxymethylcellulose sodium.

Among them, one, or any combination of two or more selected from crospovidone, croscarmellose sodium, carboxymethyl starch sodium, low substituted hydroxypropylcellulose and carboxymethylcellulose calcium is preferable.

Any compound which has been known to a person skilled in the art as an excipient may be included as the third component in the disintegrative particulate composition of the present disclosure. Typical examples of such a compound include sugars or sugar alcohols such as mannitol, erythritol, sorbitol, D-glucitol (maltitol), xylitol, trehalose, lactose and maltose. Moreover, as preferable examples thereof, mannitol, erythritol, trehalose, sorbitol and D-glucitol (maltitol) can be mentioned. As the excipient, two or more types of compounds properly selected from these compounds can also be used. Furthermore, when excipients are used in each of the first and second wet granulation steps, the excipients may be of the same type (the same combination), or may be of different types (different combinations).

The disintegrative particulate composition produced by the method of the present invention can include a crystalline cellulose known to a person skilled in the art, as the fourth component. As typical examples of such a crystalline cellulose, commercially-available products such as "Avicel" (FMC Corporation), "CEOLUS" (Asahi Kasei Chemicals Corp.), and "VIVAPUR" (RETTENMAIER) can be mentioned.

Furthermore, various types of optional components known to a person skilled in the art may properly be added and mixed into the disintegrative particulate composition of the present invention, for the purpose of adjusting various characteristics such as the disintegrating force, binding force and ease in taking the tablet, without impairing the effects of the present invention according to the above-described three or four components. As examples of such components, fluidizing agents, inorganic excipients, sweetening agents, flavoring agents and coloring agents can be mentioned.

The amount of each component blended in the disintegrative particulate composition of the present invention can properly be determined by a person skilled in the art, depending on, for example, the type of the component, the type and purpose of the medicinal ingredient, which is a target to be used in the disintegrative particulate composition, or the purpose of the final product, i.e. the orally-disintegrating tablet. In general, relative to the total weight of the disintegrative particulate composition, the amount of the first disintegrator component is within a range of 10% to 50% by weight, the amount of the second disintegrator component is within a range of 1% to 20% by weight, the amount of the crystalline cellulose, which is the fourth ingredient, is within a range of 1% to 40% by weight, and the amount of the excipient is within a range of 30% to 89% by weight.

It is preferable that the disintegrative particulate composition of the present invention have the following physical properties:

(1) an average particle size of 50 to 200 microns, e.g. 50 to 150 microns; and
(2) a water content of 0.5% to 6% by weight, e.g. 0.5% to 3% by weight.

In addition, these physical properties are measured by using the following methods and conditions.

The average particle size: 2 g of the disintegrative particulate composition is subjected to a measurement with a Φ75 mm automatic shaking sieve device (Type "M-2", Tsutsui Scientific Instruments Co., Ltd.). In addition, "R" in the present specification means a curvature radius.

The water content: 5 g of the disintegrative particulate composition is subjected to a measurement using a halogen water content measuring device (Type "HB43", METTLER TOLEDO K.K.).

There is no limitation on a specific aspect regarding the two-stage granulation for the production of the disintegrative particulate composition, and those skilled in the art may optionally select the specific conditions.

In the two-stage granulation described herein, the first and second granulation steps are carried out by a method in which each component is dispersed in the presence of water, and the dispersion is dried to form complexes, i.e. by a wet granulation process. As specific examples of the wet granulation process, spray methods such as spray drying, tumbling granulation, agitation granulation and fluidized-bed granulation, the freeze-drying method, kneading granulation, and the like can be mentioned, and the composition can be produced by any of these methods known to a person skilled in the art.

Since disintegrators such as the acid-type carboxymethylcellulose are hydrophilic, by carrying out a manipulation of applying a physical force such as by agitation or the like in the presence of water according to wet granulation, the aggregated state in the dry powder converts into a state in which particles are more dispersed. Dispersion can most easily be carried out by the fluidized-bed granulation process, spray drying, tumbling granulation, agitation granulation, etc., in which dispersion by water spraying and drying are carried out, and also, drying speeds in these methods are high. Therefore, these methods are preferable.

Among them, the fluidized-bed granulation process is a granulation method in which water, an aqueous solution including a binder, or the like is sprayed onto powder, while blowing the powder up by hot air, and, for example, adjustment of spraying conditions, etc. is easy in this method. Therefore, the fluidized-bed granulation process is the most preferable method.

In the first wet granulation step of the granulation of the present disintegrative particulate composition including three components consisting of the first disintegrator component of the acid-type carboxymethylcellulose, the second disintegrator component other than the acid-type carboxymethylcellulose and the excipient, a person skilled in the art can properly determine which two types of components among the three components are used in the first wet granulation step, depending on their types, amounts, etc. For example, the first wet granulation step can be carried out by using either the first or second disintegrator component, and the excipient.

As to specific embodiments of the first method, for example, (1) a method in which two components of a first disintegrator component (or a second disintegrator component) and an excipient are used in the first wet granulation step, and the second disintegrator component (or the first disintegrator component) is used in the second wet granulation step; (2) a method in which two components of the first and second disintegrator components are used in the first wet granulation step, and an excipient is used in the second wet granulation step; and (3) a method in which two components of the first disintegrator component (or the second disintegrator component) and an excipient are used in the first wet granulation step, and two components of the second disintegrator component (or the first disintegrator component) and an excipient are used in the second wet granulation step can be mentioned.

A granulation method of a disintegrative particulate composition including a crystalline cellulose as a forth component besides the above-described three components can take the following two embodiments:

(1) a method of producing a disintegrative particulate composition, characterized by including a first wet granulation step using any two or three of the four components, and a second wet granulation step using at least granules obtained in the first wet granulation step and the remaining one or two of the four components not used in the first wet granulation step (the second method described herein); and

(2) a method of producing a disintegrative particulate composition, characterized by including a first wet granulation step using any two of the three components other than the crystalline cellulose, a second wet granulation step using at least granules obtained in the first wet granulation step and the remaining one component not used in the first wet granulation step, and a third step of mixing the crystalline cellulose into granules obtained in the second wet granulation step (the third method described herein).

Additionally, each of the above-described four components may be used only in one granulation step. For example, only granules obtained in the first wet granulation step and the remaining component(s) not used in the first wet granulation step can be used in the second wet granulation step. Alternatively, one component can be used in a plurality of granulation steps. For example, each of components such as crystalline cellulose can be used in both the first and second wet granulation steps.

Various types of the optional components, other than the above-described four components, which can be appropriately included in the disintegrative particulate composition of the present invention and which have been known to a person skilled in the art, may be properly added in the first and/or second wet granulation step. Alternatively, these optional components may also be added and mixed in an appropriate granulation step of the third step or subsequent steps.

Furthermore, a person skilled in the art can properly determine various conditions such as the spraying speed, the supply air temperature, the exhaust temperature, and the air supply rate, depending on types or amounts of components, etc.

In both of the first wet granulation step and the second wet granulation step according to the fluidized-bed granulation process, as a medium for the spray liquid, a solvent acceptable in pharmaceuticals or foods, such as water, ethanol, methanol or acetone, can be mentioned. Alternatively, as the spray liquid, for example, an aqueous solution in which less than 10% of the component(s) for the disintegrative particulate composition is dissolved can be mentioned, and, in particular, water or such an aqueous solution is preferable.

The orally-disintegrating tablet of the present invention has excellent tablet hardness and disintegrability due to the specific structures mentioned above. As preferable values, the orally-disintegrating tablet may be characterized by having a hardness of 30 to 100 N, preferably 40 to 100 N, more preferably 50 to 100 (N), still more preferably 60 to 150 N, further more preferably 70 to 150 N, and by having a disintegration time in water of 10 to 30 seconds, preferably 10 to 24 seconds, more preferably 10 to 20 seconds.

Hereinafter, the present disclosure will more specifically be described with reference to Examples.

Examples

Example 1

(Production of a disintegrative particulate composition)

As the first wet granulation step, 280 g of mannitol (D-mannitol, Merck Ltd.) and 75 g of carmellose (NS-300, GOTOKU CHEMICAL CO., LTD.) were charged to a fluidized-bed granulator (LAB-1, Powrex Corporation), and 227 g of purified water was sprayed onto the resulting mixture at a rate of 24 g/minute to thereby granulate the mixture. Further, as the second wet granulation step, 40 g of crospovidone (Polyplasdone INF-10, ISP Japan) and 100 g of a crystalline cellulose (CEOLUS PH-101, Asahi Kasei Chemicals Corp.) were added to the resulting granules, and 300 g of purified water was sprayed thereto at 10 g/minute to thereby obtain granules (a disintegrative particulate composition of the present invention). 0.5 parts by weight of magnesium stearate (Taihei Chemical Industrial Co. Ltd.) was added to 99.5 parts by weight of the obtained granules, and these were mixed. The mixture was then subjected to tableting at tablet compression forces of 6.0 kN with a simple tableting machine (ICHIHASHI-SEIKI Co., Ltd.) to thereby obtain tablets having a diameter of 8.0 mm, R6.5, and a weight of 250 mg. In addition, the granules had the following values for physical properties: (1) an average particle size of 93 microns and (2) a water content of 1.8% by weight.

(Production of an orally-disintegrating tablet)

As the third wet granulation step, 59.5 parts by weight of the resulting disintegrative particulate composition was mixed with 40 parts by weight of clotrimazole (Wako Pure Chemical Industries, Ltd.) and 0.5 parts by weight of magnesium stearate (Taihei Chemical Industrial Co. Ltd.). The mixture was then subjected to tableting at a tablet compression force of 6.0 kN with the simple tableting machine (HANDTAB-100, ICHIHASHI-SEIKI Co., Ltd.) to thereby obtain an angled-corner flat tablet having a diameter of 8.0 mm and a weight of 250 mg.

Examples 2-19

(Production of an orally-disintegrating tablet)

The disintegrative particulate composition obtained in Example 1 was mixed with the medicinal ingredient listed in Table 2 and 0.5 parts by weight of magnesium stearate (Taihei Chemical Industrial Co. Ltd.). The mixture was then subjected to tableting at a tablet compression force shown in Table 2 with the simple tableting machine (HANDTAB-100, ICHIHASHI-SEIKI Co., Ltd.) to thereby obtain an angled-corner flat tablet having a diameter of 8.0 mm and a weight of 250 mg.

[Evaluation on hardness and disintegrability tests]

Values of the physical properties of the resulting tablets were measured based on the following conditions/-methods. The test results of hardness and disintegration time are shown in Table 2.

Hardness: a hardness (N) was measured with a Kiya hardness tester (KHT-40N, Fujiwara Scientific Company Co., Ltd.) .

Disintegration time in water: a disintegration time in water was measured with a disintegration tester (NT-4HF, TOYAMA SANGYO CO., LTD.) in accordance with the method described in the Japanese Pharmacopoeia provided that an auxiliary disk was not used. However, the auxiliary disk was used for the tablets comprising bicarbonates.

The measurements for the hardness and disintegration time were each repeated six times, and average values thereof were regarded as measurement results.

[Table 2]

|  | Medicinal Ingredient | Calculated LogP | | Content (parts by weight) | Tablet Compression Force (kN) | Tablet Hardness (N) | Disintegration Time in Water (s) |
|---|---|---|---|---|---|---|---|
| Disintegrative Particulate Composition |  |  |  | 0 | 6 | 74 | 13 |
| Example 1 | clotrimazole | 5.19 | 1) | 40 | 6 | 80 | 18 |
| Example 2 |  |  |  | 50 | 6 | 84 | 21 |
| Example 3 | ibuprofen | 3.83 | 1) | 40 | 8 | 57 | 15 |
| Example 4 |  |  |  | 50 | 8 | 59 | 16 |
| Example 5 |  |  |  | 10 | 8 | 53 | 18 |
| Example 6 |  |  |  | 80 | 15 | 49 | 19 |
| Example 7 | ethenzamide | 0.97 | 1) | 40 | 8 | 11 | 13 |
| Example 8 |  |  |  | 50 | 8 | 12 | 15 |
| Example 9 |  |  |  | 60 | 10 | 67 | 11 |
| Example 10 | acetaminophen | 0.61 | 1) | 40 | 12 | 63 | 14 |
| Example 11 |  |  |  | 50 | 12 | 46 | 13 |
| Example 12 | famotidine | -0.42 | 1) | 40 | 8 | 18 | 25 |
| Example 13 |  |  |  | 50 | 8 | 13 | 20 |
| Example 14 | ascorbic acid | -1.88 | 2) | 40 | 10 | 54 | 13 |
| Example 15 |  |  |  | 50 | 14 | 50 | 22 |
| Example 16 | glycine | -3.41 | 2) | 40 | 10 | 62 | 13 |
| Example 11 |  |  |  | 50 | 12 | 53 | 13 |
| Example 18 | aspartic acid | -4.32 | 2) | 40 | 12 | 59 | 14 |
| Example 19 |  |  |  | 50 | 12 | 44 | 14 |
| 1) LogP Values calculated by CAChe 2) LogP Values calculated by KOWWIN | | | | | | | |

Industrial Applicability

The present invention significantly contributes to research and development of orally-disintegrating tablets having excellent tablet hardness and disintegrability.

**Claims**

**1.** An orally disintegrating tablet comprising a medicinal ingredient having distribution coefficient (logP) in a range of from -5.0 to 6.0 and a disintegrative particulate composition,

   wherein the content of the medicinal ingredient is 40% by weight or more;

wherein the disintegrative particulate composition includes four components consisting of a first disintegrator component of an acid-type carboxymethylcellulose, a second disintegrator component other than the acid-type carboxymethylcellulose, an excipient of sugars or sugar alcohols, and crystalline cellulose;

wherein the disintegrative particulate composition is produced by a two-stage granulation comprising a first wet granulation step using any two or three of the four components, and a second wet granulation step using at least granules obtained in the first wet granulation step and the remaining one or two of the four components not used in the first wet granulation step; and

wherein the distribution coefficient is calculated according to the formulation (1) when the medicinal ingredient does not have a structure of a zwitterion, and to formulation (2) when the medicinal ingredient has a structure of a zwitterion:

(1) CAChe using the following equation:

$$\texttt{LogP = \Sigma niai}$$

wherein "ni" means the number of each group of atoms, and "ai" means a contribution ratio of the group of atoms;

(2) KOWWIN using the following equation:

$$\texttt{LogP = \Sigma fini + \Sigma cjnj + 0.229}$$

wherein "fi" means the coefficient of each group of atoms, and "ni" means the number of the group of atoms, and "cj" means the coefficient of each correction factor, and "nj" means the number of the correction factor.

2. The orally disintegrating tablet according to Claim 1,
wherein the second disintegrator component is one or any combination of two or more selected from crospovidone, croscarmellose sodium, carboxymethyl starch sodium, low substituted hydroxypropylcellulose and carboxymethylcellulose calcium.

3. The orally disintegrating tablet according to Claim 2,
wherein the second disintegrator component is crospovidone.

4. The orally disintegrating tablet according to any one of Claims 1 to 3, wherein the sugars or sugars alcohol are mannitol, erythritol, sorbitol, D-glucitol (maltitol), xylitol, trehalose, lactose or maltose.

5. The orally disintegrating tablet according to Claim 4,
wherein the sugars or sugar alcohols are mannitol.

6. The orally disintegrating tablet according to Claim 1,
wherein the content of the medicinal ingredient is 50% or more.

7. The orally disintegrating tablet according to Claim 6,
wherein the distribution coefficient (logP) of the medicinal ingredient is in a range of from 0.8 to 4.0 and the content of the medicinal ingredient is 60% or more.

**Patentansprüche**

1. Oral zerfallende Tablette, umfassend einen medizinischen Inhaltsstoff mit einem Verteilungskoeffizienten (logP) in einem Bereich von -5,0 bis 6,0 und eine zerfallende teilchenförmige Zusammensetzung,

wobei der Gehalt des medizinischen Inhaltsstoffs 40 Gew.-% oder mehr beträgt;
wobei die zerfallende teilchenförmige Zusammensetzung vier Komponenten beinhaltet, die aus einer ersten Zerkleinererkomponente einer Carboxymethylcellulose des sauren Typs, einer zweiten Zerkleinererkomponente, die nicht die Carboxymethylcellulose des sauren Typs ist, einem Hilfsstoff aus Zuckern oder Zuckeralkoholen und kristalliner Zellulose besteht;
wobei die zerfallende teilchenförmige Zusammensetzung durch eine zweistufige Granulierung hergestellt wird, die einen ersten Nassgranulationsschritt unter Verwendung von zwei oder drei der vier Komponenten und einen zweiten Nassgranulationsschritt unter Verwendung von wenigstens Granulat, das in dem ersten Nassgranulationsschritt erhalten wurde, und der verbleibenden einen oder zwei der vier Komponenten umfasst, die in dem ersten Nassgranulierungsschritt nicht verwendet wurden; und

wobei der Verteilungskoeffizient gemäß der Formulierung (1) berechnet wird, wenn der medizinische Inhaltsstoff keine Struktur eines Zwitterions aufweist, und gemäß der Formulierung (2) berechnet wird, wenn der medizinische Inhaltsstoff eine Struktur eines Zwitterions aufweist:

(1) CAChe unter Verwendung der folgenden Gleichung:

$$LogP = \Sigma n_i a_i$$

wobei "ni" die Anzahl jeder Gruppe von Atomen bedeutet und "ai" ein Beitragsverhältnis der Gruppe von Atomen bedeutet;
(2) KOWWIN unter Verwendung der folgenden Gleichung:

$$LogP = \Sigma f_i n_i + \Sigma c_j n_j + 0{,}229$$

wobei "fi" den Koeffizienten jeder Gruppe von Atomen bedeutet, "ni" die Anzahl der Atome in der Gruppe der Atome bedeutet und "cj" den Koeffizienten jedes Korrekturfaktors bedeutet und "nj" die Anzahl des Korrekturfaktors bedeutet.

2. Oral zerfallende Tablette nach Anspruch 1, wobei die zweite Zerkleinererkomponente eine oder eine beliebige Kombination von zwei oder mehr Komponenten ist, ausgewählt aus Crospovidon, Croscarmellosenatrium, Carboxymethylstärkenatrium, niedrig substituierter Hydroxypropylcellulose und Carboxymethylcellulosekalzium.

3. Oral zerfallende Tablette nach Anspruch 2, wobei die zweite Zerkleinererkomponente Crospovidon ist.

4. Oral zerfallende Tablette nach einem der Ansprüche 1 bis 3, wobei die Zucker oder Zuckeralkohole Mannit, Erythrit, Sorbit, D-Glucitol (Maltit), Xylit, Trehalose, Lactose oder Maltose sind.

5. Oral zerfallende Tablette nach Anspruch 4, wobei die Zucker oder Zuckeralkohole Mannit sind.

6. Oral zerfallende Tablette nach Anspruch 1, wobei der Gehalt an dem medizinischen Inhaltsstoff 50 % oder mehr beträgt.

7. Oral zerfallende Tablette nach Anspruch 6, wobei der Verteilungskoeffizient (logP) des medizinischen Inhaltsstoffs in einem Bereich von 0,8 bis 4,0 liegt und der Gehalt des medizinischen Inhaltsstoffs 60 % oder mehr beträgt.

**Revendications**

1. Comprimé orodispersible comprenant un ingrédient médicinal ayant un coefficient de distribution (logP) compris dans une plage allant de -5,0 à 6,0 et une composition particulaire dispersible,

dans lequel la teneur en ingrédient médicinal est supérieure ou égale à 40 % en poids ;
dans lequel la composition particulaire dispersible comporte quatre composants consistant en un premier composant dispersible consistant en une carboxyméthylcellulose de type acide, un deuxième composant dispersible autre que la carboxyméthylcellulose de type acide, un excipient consistant en des sucres ou alcools de sucre, et une cellulose cristalline ;
dans lequel la composition particulaire dispersible est produite par granulation à deux étages comprenant une première étape de granulation humide mettant en oeuvre deux ou trois des quatre composants, et une deuxième étape de granulation humide mettant en oeuvre au moins des granulés obtenus lors de la première étape de granulation humide et l'un ou les deux composants restants desdits quatre composants qui n'avaient pas été mis en oeuvre lors de la première étape de granulation humide ; et
dans lequel le coefficient de distribution est calculé selon la formulation (1) lorsque l'ingrédient médicinal ne présente pas de structure zwitterionique, et selon la formulation (2) lorsque l'ingrédient médicinal présente une structure zwitterionique :

(1) CAChe en mettant en oeuvre l'équation suivante :

$$LogP = \Sigma n_i a_i$$

où « ni » représente le nombre de chaque groupe d'atomes, et « ai » représente le rapport de contribution du groupe d'atomes ;
(2) KOWWIN en mettant en oeuvre l'équation suivante :

$$LogP = \Sigma f_i n_i + \Sigma c_j n_j + 0,229$$

où « fi » représente le coefficient de chaque groupe d'atomes, « ni » représente le nombre du groupe d'atomes, « cj » représente le coefficient de chaque facteur de correction, et « nj » représente le nombre du facteur de correction.

2. Comprimé orodispersible selon la revendication 1, dans lequel le deuxième composant dispersible consiste en un composant ou une quelconque combinaison d'au moins deux composants sélectionnés parmi la crospovidone, la croscarmellose sodique, le carboxyméthylamidon sodique, l'hydroxypropylcellulose faiblement substituée et la carboxyméthylcellulose calcique.

3. Comprimé orodispersible selon la revendication 2, dans lequel le deuxième composant dispersible est la crospovidone.

4. Comprimé orodispersible selon l'une quelconque des revendications 1 à 3, dans lequel les sucres ou l'alcool de sucres sont le mannitol, l'érythritol, le sorbitol, le D-glucitol (maltitol), le xylitol, le tréhalose, le lactose ou le maltose.

5. Comprimé orodispersible selon la revendication 4, dans lequel les sucres ou alcools de sucre sont le mannitol.

6. Comprimé orodispersible selon la revendication 1, dans lequel la teneur en ingrédient médicinal est supérieure ou égale à 50 %.

7. Comprimé orodispersible selon la revendication 6, dans lequel le coefficient de distribution (logP) de l'ingrédient médicinal est compris dans une plage allant de 0,8 à 4,0 et la teneur en ingrédient médicinal est supérieure ou égale à 60 %.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011019045 A
- JP 2008285434 A
- JP 2012031138 A
- JP 4551627 B
- EP 2832350 A1
- EP 2898899 A1
- EP 2465539 A1
- EP 1923074 A1

### Non-patent literature cited in the description

- *Journal of Computational Chemistry*, 1988, vol. 9 (1), 80-90
- *Journal of Pharmacological Science*, 1995, vol. 84, 83-92